# EUROPEAN PATENT SPECIFICATION

(11) **EP 1 561 418 B1**
(45) Date of publication and mention of the grant of the patent: **17.08.2011**
(21) Application number: 05002028.8
(22) Date of filing: 01.02.2005
(51) Int. Cl.: A61B 5/00

(54) **Analyzer, cartridge, cartridge kit**
Analysegerät, Patrone, Patronenkit
Analyseur, cartouche, kit à cartouche

(30) Priority: 03.02.2004 JP 2004026483
(43) Date of publication of application: 10.08.2005
(73) Proprietor: SYSMEX CORPORATION, Kobe-shi, Hyogo 651-0073 (JP)
(72) Inventor: Sawa, Kenichi, Amagasaki-shi Hyogo 661-0024 (JP); Maekawa, Yasunori, Kobe 651-2277 (JP); Sato, Toshiyuki, Nishinomiya-shi Hyogo 662-0024 (JP); Nagaoka, Kanako, Kobe 654-0031 (JP); Okada, Seiki, Kobe-shi Hyogo 651-0079 (JP); Hagino, Kei, Kobe 651-2273 (JP)
(74) Representative: HOFFMANN EITLE

(56) References cited:
- WO-A-00/74763
- US-A1- 2003 143 746

## Description

### FIELD OF THE INVENTION

The present invention relates to an analyzer, cartridge and cartridge kit.

### BACKGROUND

Devices for extracting glucose through the skin using the reverse iontophoretic method (for example, International Patent Publications WO9600110 and WO9710356) are known as transdermal analyte extraction assemblies for extracting the glucose from the interstitial fluid of living tissue.

These devices include a gel which is provided beforehand in a reservoir. The gel includes an enzyme, such as glucose oxidase (GOD) or the like, which is a catalyst for converting the glucose to gluconic acid and hydrogen peroxide. These devices measure glucose concentration by detecting the electrical charge generated by converting the glucose.

These devices, however, have problems inasmuch as the gel and the like is provided beforehand in a reservoir, and the water in the gel evaporates before the assembly is used such that the assembly cannot be used any more. Accordingly, the assemblies are difficult to store for long periods.

Furthermore, since enzymes, such as GOD and the like, deteriorate in a short time by contacting with moisture, these assemblies have poor storage stability, and are difficult to store for long periods.

The International Patent Publication WO9600110 even proposes to stabilize the constituents of the conductive medium by using a dry-type conductive medium, such as dehydrating gel, and adding water or electrolyte before mounting the conductive medium on the skin. However, the mechanism for adding the water is not described in International Patent Publication W09600110. Furthermore, International Patent Publication WO9600110 requires adding water to two assemblies, thereby creating the problem of complex operation.

WO00/74763 discloses a microneedle device for withdrawing and sensing biological fluids through the skin. The preamble of the independent claims is based on this document. US 2003/0143746 discloses a body analyte monitoring system.

### SUMMARY

The scope of the present invention is defined solely by the appended claims, and is not affected to any degree by the statements within this summary.

An object of the present invention is to provide a novel analyzer and cartridge which solve these problems.

A first aspect of the present invention relates to an analyzer for extracting and analyzing an analyte from a subject, the analyzer comprising: a reservoir capable of holding a liquid for holding the analyte extracted from the subject; and a liquid supplying mechanism for holding a liquid to be supplied to the reservoir and supplying the liquid to the reservoir.

A second aspect of the present invention relates to a cartridge detachably mountable to an analyzer for extracting and analyzing an analyte from a subject, the cartridge comprising: a reservoir capable of holding a liquid for holding the analyte extracted from the subject; and a liquid supplying mechanism for holding a liquid to be supplied to the reservoir and supplying the liquid to the reservoir.

An enzyme is held in a dry state by the reservoir. The enzyme is a catalyst for the analyte.

Disclosed herein is an analyzing method for extracting an analyzing an analyte from a subject using an analyzer which includes an enzyme holder for holding an enzyme which is a catalyst for the analyte extracted from the subject, the method comprising: (a) a step of bringing the enzyme held in a dry state in the enzyme holder into contact with a holding substance for holding the analyte extracted from the subject; (b) a step of placing the analyzer on the subject; (c) a step of extracting analyte from the subject to the holding substance; and (d) a step of analyzing the analyte extracted to the holding substance.

### BRIEF DESCRIPTION OF THE DRAWINGS

Fig. 1(a) is a partial broken-out top view of a biological component analyzer, and Fig. 1(b) is a cross-section view illustrating the biological component analyzer of 1(a) mounted on the wrist of a user by means of a band;
Fig. 2 is a top plan view of the cartridge housing of the biological component extraction cartridge of Fig. 1;
Fig. 3(a) is a cross section view on the A-A line of Fig. 2, (b) is a cross section view on the B-B line of Fig. 2, and (c) is a cross section view on the C-C line of Fig. 2;
Fig. 4 is an enlarged section view on the P-P line of Fig. 1;
Fig. 5 is a cross section view of the sensor member mounted on the biological component extraction cartridge of the present embodiment;
Fig. 6(a) is a partial broken-out top view of a biological component analyzer of an embodiment of the present invention, and Fig. 6(b) is a cross-section view illustrating the biological component analyzer of 1(a) mounted on the wrist of a user by means of a band;
Fig. 7 is a top plan view of the cartridge housing of the biological component extraction cartridge of Fig. 6;
Fig. 8 is an enlarged section view on the Q-Q line of Fig. 6(a);
Fig. 9 is a top view of the cartridge housing used in the biological component analyzer of still another embodiment of the present invention;
Fig. 10 is a cross section view on the A-A line of Fig. 9;
Fig. 11 illustrates the insertion device of another embodiment of the present invention;
Fig. 12 is a flow chart of the analyzing method of another embodiment of the present invention;
Fig. 13 is a schematic view of a cross section of the extraction region of the skin in which extraction holes are formed;
Fig. 14 is a schematic view of a cross section of the extraction region of the skin in which physiological saline flows into the extraction holes;
Fig. 15 is a schematic view of a cross section of the extraction region of the skin in which fluid secreted from the corium into the extraction holes is diffused in the physiological saline;
Fig. 16 is a schematic view of a cross section of the extraction region of the skin in which fluid extracted into the saline solution in the extraction holes is moved toward the extraction unit by an applied electric field; and
Fig. 17 is a perspective view showing a container for supplying the physiological saline to a cartridge of another embodiment of the present invention.

### DETAILED DESCRIPTION OF THE PREFERRED EMBODIMENTS

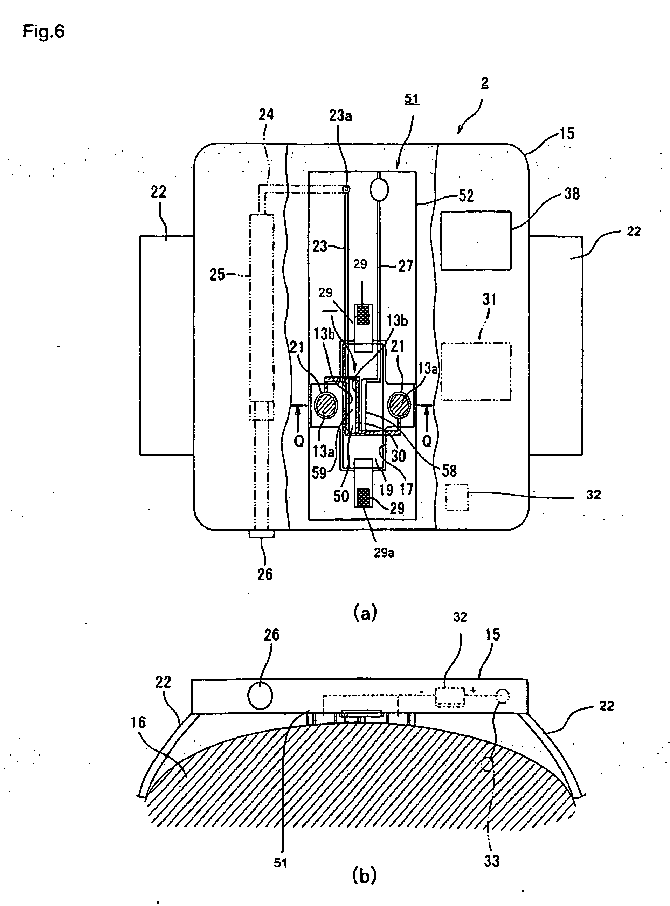

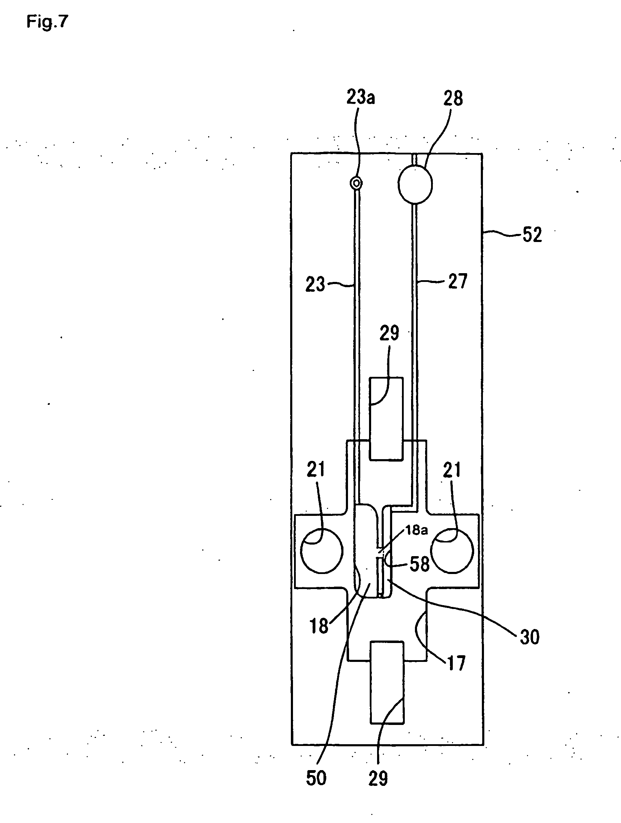

A preferred embodiment of the present invention is described hereinafter with reference to Figures 6 to 8 of the drawings.

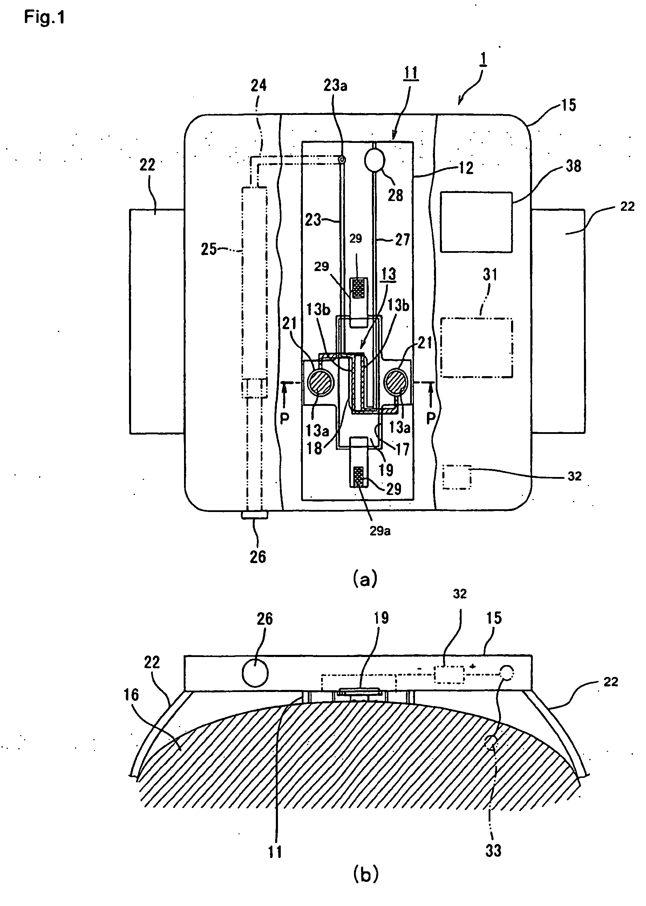

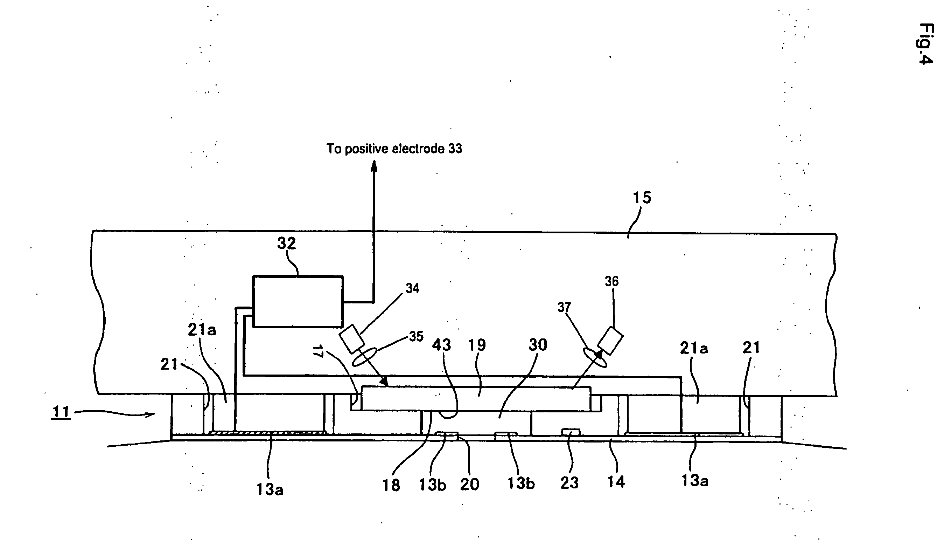

Fig. 1(a) is a partial broken-out top view of a biological component analyzer 1 of an embodiment not belonging to the present invention. This embodiment of the biological component analyzer 1 is a device for analyzing glucose, which is one type of biological component, and is provided with a biological component extraction cartridge 11, and an analyzing unit 15 on the bottom side (skin side) of which the biological component extraction cartridge 11 is detachably secured. The biological component extraction cartridge 11 includes a cartridge housing 12 formed of resin such as acrylic or the like, extraction electrode 13 (shaded area) for extracting glucose, sensor member 19, and containment membrane 14 (Fig. 4) described later. Two mounting holes 29 are provided in the cartridge housing 12. The analyzing unit 15 is provided with two hooks 29a positioned on the underside in correspondence with the mounting holes 29. The two hooks 29a are forced in directions that widen their mutual spacing. The biological component extraction cartridge 11 is detachably mounted in the analyzing unit 15 by inserting the hooks 29a into the two mounting holes 29 so as to be supportably held by the force exerted by the two hooks 29a. The biological component extraction cartridge 11 is replaced each time glucose is measured. The analyzing unit 15 is provided with band 22 for fixing the analyzing unit 15 to the wrist 16 of a user, a flow path 24, syringe pump 25 which supplies physiological saline to the biological component extraction cartridge 11 through the flow path 24and which is detachably installed in the analyzing unit 15, controller 31 for calculating the amount of glucose and calculating a blood sugar value based on the calculated amount of glucose, constant-voltage power supply 32, and a display 38 for displaying the calculated amount of glucose and blood sugar value. The controller 31 is provided with a microcomputer including a CPU, ROM, RAM and the like, and analog/digital conversion circuits and the like. Fig. 1(b) is a cross section view of the biological component analyzer 1 mounted on the wrist 16 of a user by means of a band 22; the biological component analyzer 1 is mounted so as to have the bottom surface of the biological component extraction cartridge 11 in close contact with the skin of wrist 16 of the user.

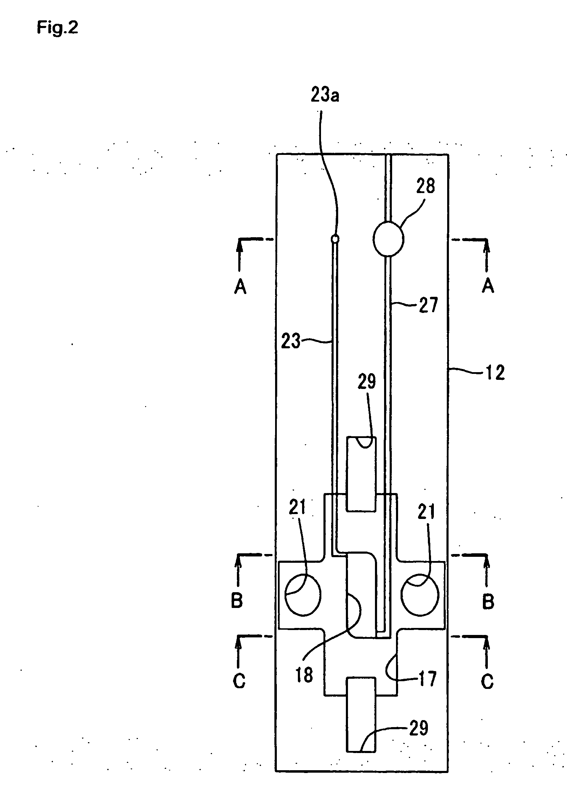

The biological component analyzer 1 is described below referring to Figs. 2 through 4. As shown in Fig. 2, a cross-shaped concavity 17 for accommodating lower side of the sensor member 19 is provided in the cartridge housing 12.The cartridge housing 12 has a rectangle-shaped through-hole passing through from the analyzing unit 15 side (top side) to the skin side (bottom side) of the cartridge housing 12 in the approximate center of the concavity 17. The opening of the through-hole on the analyzing unit 15 side is designated first opening 18, and the opening on the skin side is designated second opening 20 (refer to Fig. 4). The lower side of the sensor member 19 is arranged in the concavity 17 so as to block the first opening 18. The part of the bottom surface of the sensor member 19 exposed to the skin through the first opening 18 is a measuring surface 43. A gel containing an enzyme (glucose oxidase (GOD)) which is a catalyst for glucose, an enzyme (peroxidase (POD)) which is a catalyst for hydrogen peroxide (H₂O₂) produced from glucose in the presence of GOD, and a luminescence-producing reagent (N,N-bis(2-hydroxy-3-sulfopropyl)tolidene dipotassium salt) that reacts with activated oxygen (O*) produced from H₂O₂ in the presence of POD is applied to the measuring surface 43, and this gel is subjected to a drying process.

The containment membrane 14 is a sheet the same size as the bottom surface (skin side surface) of the cartridge housing 12, and has an opening smaller than the opening 20 positioned opposite the opening 20.

The containment membrane 14 is adhered to the bottom surface of the cartridge housing 12, so as to cover the entire bottom surface of the cartridge housing 12 excluding the center area of the second opening 20.

Two working parts 13b of the extraction electrode 13 are disposed on the top surface of the containment membrane 14 in the area exposed to the analyzing unit 15 side from the second opening 20, so as to interpose the second opening 20 therebetween (refer to Fig. 4). A reaction reservoir 30 is a chamber formed by blocking the through-hole formed in the center of the concavity 17 by part of the containment membrane 14 and the sensor member 19. The luminescence-producing reagent and other enzymes such as GOD and POD are retained in the reaction reservoir 30 by application to the measuring surface 43 exposed to the inside of the reaction reservoir 30.

The extraction electrode 13 has two round terminals 13a at the bilateral ends of the biological component extraction cartridge 11, and two center working parts 13b respectively connected to the terminals 13a, as indicated by the oblique shading in Fig. 1. The extraction electrode 13 is formed by punching a plate from a conductive material such as metal, and depositing the containment membrane 14 on its top surface. As shown in Fig. 1, the two terminals 13a are respectively housed in two fixing holes 21 provided in the cartridge housing 12, and are anchored by fixing members 21a, as shown in Fig. 4. The two terminals 13a of the extraction electrode 13 are connected to the negative side of the constant-voltage power supply 32 provided in the analyzing unit 15, and a current is supplied from the negative side of the constant-voltage power supply 32 to extract glucose through the skin of the subject. Furthermore, the positive side of the constant-voltage power supply 32 is connected to a positive electrode 33 which is electrically connected to the skin of the subject, as shown in Fig. 1(b).

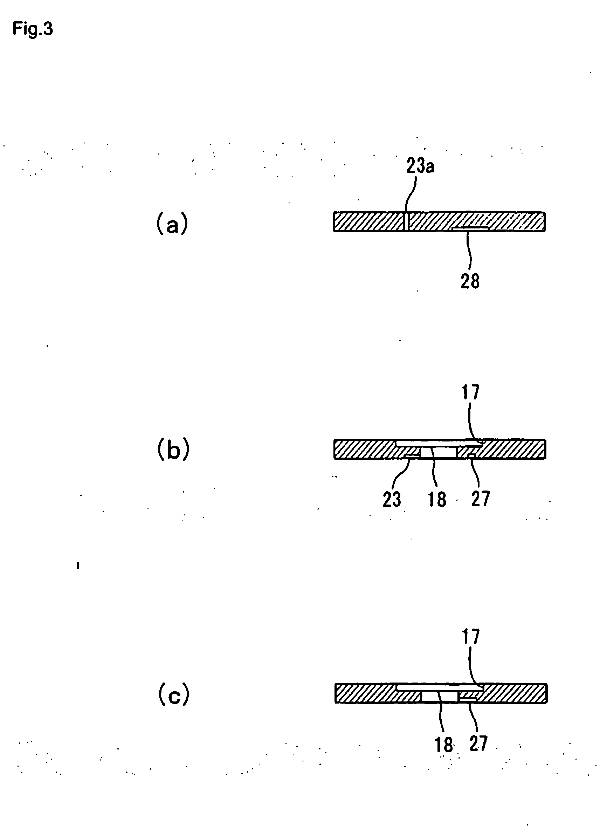

As shown in Fig. 2, a flow path 23 is provided which extends from the reaction reservoir 30 in a mainly vertical direction of the cartridge housing 12 in the cartridge housing 12. The flow path 23 forms a tube-like path having a square cross section by forming a channel on the bottom surface of the cartridge housing 12 and blocking the bottom surface with the containment membrane 14, as shown in Fig. 3(b) and Fig. 4. A connector hole 23a at the top end of the flow path 23 is connected to the syringe pump 25 in the analyzing unit 15 through the flow path 24, as shown in Figs. 2 and 3(a). The syringe pump 25 holds a quantity of physiological saline sufficient for several measurements, and an amount of physiological saline required to fill the reaction reservoir 30 is supplied to the reaction reservoir 30 through the flow path 24 and flow path 23 by pressing the piston 26. The physiological saline supplied to the reaction reservoir 30 is held by the reaction reservoir 30 and holds the glucose extracted from the subject, and supplies the glucose to the measuring surface 43.

As shown in Fig. 2, a discharge path 27 is provided which extends from the reaction reservoir 30 in a mainly vertical direction parallel to the flow path 23 in the cartridge housing 12. The discharge path 27 is formed by a tube-like channel with a square cross section on the bottom surface of the cartridge housing 12, the bottom surface of which is blocked by the containment membrane 14, as shown in Figs. 3(b) and 3(c). The discharge path 27 opens to the exterior of the device (atmosphere) through a buffer space 28.

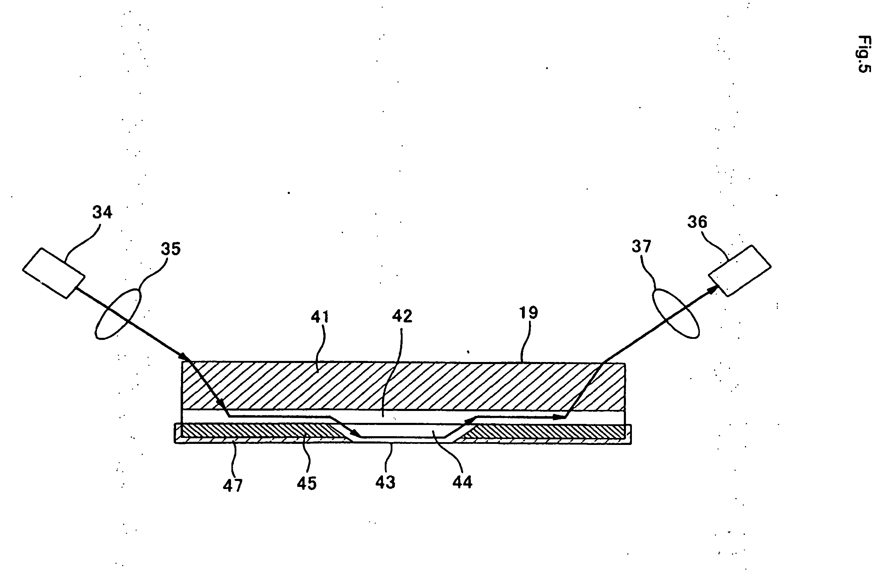

Fig. 5 is a cross section view of the sensor member 19 mounted on the biological component extraction cartridge 11 of the present embodiment. The sensor member 19 has a glass substrate 41, and a first optical waveguide 42 having a high refractive index is formed on the front surface of the substrate 41 on the skin side. A second optical waveguide 44, which has a refractive index higher than the first optical waveguide 42, has a exterior with an inclined configuration and is formed the surface on the skin side at the center of the first optical waveguide 42. A protective layer 45 is formed on the areas of the surface on the skin side of the first optical waveguide 42 where the second optical waveguide 44 is not formed, and the surface of the protective layer 45 on the skin side is covered by a light-blocking layer 47. The part of the second optical waveguide 44 which is exposed on the skin side is the measuring surface 43.

As shown in Figs. 4 and 5, the analyzing unit 15 is provided with a monochromatic light source 34, and a lens 35 for guiding the light from the monochromatic light source 34 through the substrate 41 to the first optical waveguide 42. The light directed to the first optical waveguide 42 enters the second optical waveguide 44, and is completely and repeatedly reflected within the second optical waveguide 44 and directed back to the first optical waveguide 42, and enters a photoreceptor element 36 through the substrate 41 and a lens 37. The lens 37 and photoreceptor element 36 are also provided in the analyzing unit 15. The light received by the photoreceptor element 36 is converted to electrical signals by the photoreceptor element 36 in accordance with the amount of light, and input to the controller 31.

The sensor member 19 is arranged such that the measuring surface 43 is aligned with the first opening 18 of the cartridge housing 12.

When the glucose extracted from the subject is delivered to the measuring surface 43 through the physiological saline supplied from the syringe pump 25 to the reaction reservoir 30, the GOD applied to the measuring surface 43 acts as a catalyst in reaction with the glucose to produce H2O2 and gluconic acid. Then, the POD applied to the measuring surface 43 acts as a catalyst in reaction with the H2O2 to produce activated oxygen (O*) and water (H2O). Next, luminescence occurs when the activated oxygen reacts with the luminescence -producing reagent applied to the measuring surface 43. When the light transits within the second optical waveguide 44, the light changes the angle of reflection by the luminescence. Then the light reaches the photoreceptor 36. Accordingly, the electrical signal output from the photoreceptor 36 is of a magnitude which reflects the amount of glucose that reached the measuring surface 43.

Pyranose oxidase, hexokinase, glucokinase, glucose dehydrogenase and the like may be used rather than the glucose oxidase mentioned above. Furthermore, 3,3',5,5'-tetramethylbenzylidene may be used alternatively to the N,N-bis(2-hydroxy-3-sulfopropyl)tolidene dipotassium salt as the luminescence -producing reagent.

The biological component analyzer 1 of the embodiment described above is used as follows. First, the biological component extraction cartridge 11 is loaded in the analyzing unit 15, and the biological component analyzer 1 is attached to the wrist of the user by the band 22 such that the containment membrane 14 on the bottom surface of the biological component extraction cartridge 11 is in contact with the skin of the user. Then, physiological saline is expressed from the syringe pump 25 through the flow path 24 and flow path 23 by pressing the piston 26, and the physiological saline fills the reaction reservoir 30. Next, glucose is extracted through the skin of the subject to the physiological saline in the reaction reservoir 30 by applying a predetermined voltage (2 V) from the constant-voltage power supply 32 to the extraction electrode 13 for a predetermined time (3 minutes). The glucose extracted to the physiological saline within the reaction reservoir 30 migrates within the physiological saline and reaches the measuring surface 43.

Then, as previously described, the GOD acts as a catalyst and luminescence occurs. The amount of light at this time is detected by the photoreceptor 36, and an electrical signal corresponding to the amount of detected light is input to the controller 31. The controller 31 calculates the glucose concentration based on the received signal, and a blood sugar value is calculated from the calculated glucose concentration. The calculated glucose concentration and blood sugar value are displayed on the display unit 38.

Although the physiological saline is supplied from the syringe pump 25 to the reaction reservoir 30 after the biological component analyzer 1 is mounted on the wrist of the user in the present embodiment, the present invention is not limited to this mode inasmuch as the physiological saline may be supplied from the syringe pump 25 to the reaction reservoir 30 before the biological component analyzer 1 is mounted on the wrist of the user, and the analyzer 1 may thereafter be mounted on the wrist of the user.

In the biological component analyzer 1 of the present embodiment, inactivation of the enzyme by moisture is prevented and the biological component extraction cartridge 11 can be stored for a long time because the enzymes and physiological saline are stored separately and the gel containing the enzymes is dried when the biological component extraction cartridge 11 is not used. Furthermore, since the biological component analyzer 1 of the present embodiment uses a liquid such as physiological saline, the liquid can be in close contact with the skin of the user. Consequently, the amount of glucose extracted from the subject is stable and analysis accuracy is improved. In addition, the biological component extraction cartridge 11 of the present embodiment can be used by supplying physiological saline from the syringe pump 25 to the reaction reservoir 30 even after the cartridge has been stored for a long period. Accordingly, it is possible to store the cartridge for longer periods compared to the conventional art. The biological component analyzer 1 is capable of extracting glucose by means of a simple operation when a single biological component extraction cartridge 11 is loaded in the analyzing unit 15.

The biological component analyzer 2 of an embodiment of the present invention is described below with reference to Figs. 6 through 8. Whereas the biological component analyzer 1 of the previous embodiment performed glucose extraction and detection in the reaction reservoir 30, the biological component analyzer 2 of the present embodiment differs from the biological component analyzer 1 mainly in that a separate extraction reservoir 50 is provided in addition to the reaction reservoir 30. In Figs. 6 through 8 of the present embodiment, parts in common with Figs. 1 through 5 are designated by the same reference numbers.

In the biological component analyzer 2, a first concavity 58 is formed at the approximate center within a concavity 17 of a cartridge housing 52. The measuring surface 43 of the sensor member 19 is arranged at the opening of the first concavity 58. Accordingly, in the biological component analyzer 2, the reaction reservoir 30 is formed by blocking the first concavity 58 with the sensor member 19.

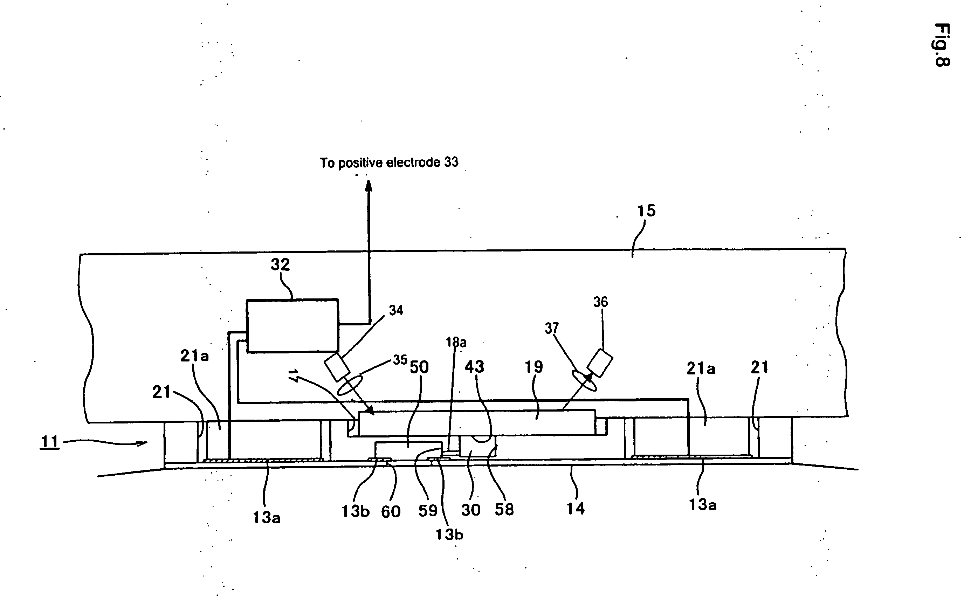

The biological component analyzer 2 has an extraction concavity 59 provided on the bottom side of the cartridge housing 52 adjacent to the first concavity 58 (refer to Fig. 8).

The containment membrane 14 is a seal the same size as the bottom surface of the cartridge housing 52, and has an opening smaller than the opening of the extraction concavity 59 positioned at the opening of the extraction concavity 59.

The containment membrane 14 is adhered to the bottom surface of the cartridge housing 52, so as to cover the entire bottom surface of the cartridge housing 52 excluding the part of the opening of the extraction concavity 59.

Two working parts 13b of the extraction electrode 13 are disposed on the top surface of the containment membrane 14 in the area exposed to the analyzing unit 15 side from the opening of the extraction concavity 59, so as to interpose the opening therebetween.

A extraction reservoir 50 is a chamber formed by the extraction concavity 59, and the containment membrane 14 which covers part of the opening. The sensor member 19 is not exposed within the extraction concavity 59. The reaction reservoir 30 and extraction reservoir 50 are connected by a flow path 18a. The reaction reservoir 30 is open to the outside (atmosphere) through a discharge path 27 and buffer space 28. The extraction reservoir 50 is connected to the syringe pump 25 through the flow path 23 and flow path 24.

In the biological component analyzer 2 of the present embodiment, glucose analysis is performed as described below. First, a biological component extraction cartridge 51 is loaded in the analyzing unit 15, and the analyzer 2 is attached to the wrist of the user by the band 22 such that the containment membrane 14 on the bottom surface of the extraction cartridge 51 is in close contact with the skin of the user. Then, physiological saline is expressed from the syringe pump 25 through the flow path 24 and flow path 23 by pressing the piston 26. Consequently, the saline solution fills the extraction reservoir 50. Next, the glucose is extracted through the skin of the subject to the physiological saline in the extraction reservoir 50 by applying a predetermined voltage (2 V) from the constant-voltage power supply 32 to the extraction electrode 13 for a predetermined time (3 minutes). Then, the piston 26 is operated again and the physiological saline containing the extracted glucose within the extraction reservoir 50 is moved to the reaction reservoir 30. In the reaction reservoir 30, the GOD applied to the measuring surface 43 acts as a catalyst and luminescence occurs as previously described, and an electrical signal reflecting the amount of glucose is output from the photoreceptor element 36 as previously described. The controller 31 calculates the glucose concentration based on the received signal, and a blood sugar value is calculated from the calculated glucose concentration. The calculated glucose concentration and blood sugar value are displayed on the display unit 38.

Although the above two devices have been described in terms of the physiological saline moving to the reaction reservoir 30 and the extraction reservoir 50 by the user operating the piston 26, the present invention is not limited to this mode inasmuch as the present invention may also be applied to biological component analyzers constructed so as to move physiological saline to the reaction reservoir 30 or extraction reservoir 50 by the automatic operation of a pump when the user presses a switch to start the measurement. A measurement start switch 26 also may be provided on a side surface of the analyzing unit 15, or near the display 33 or the like.

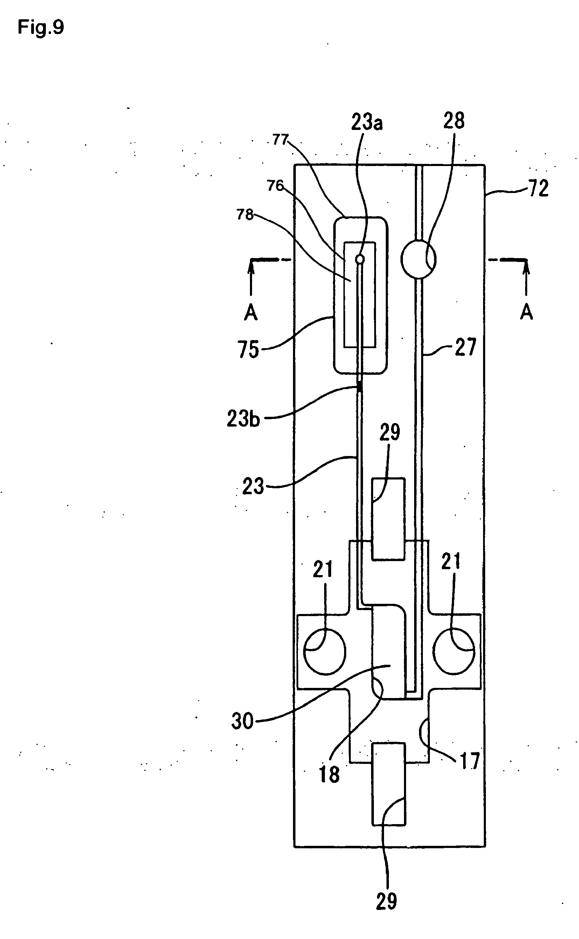

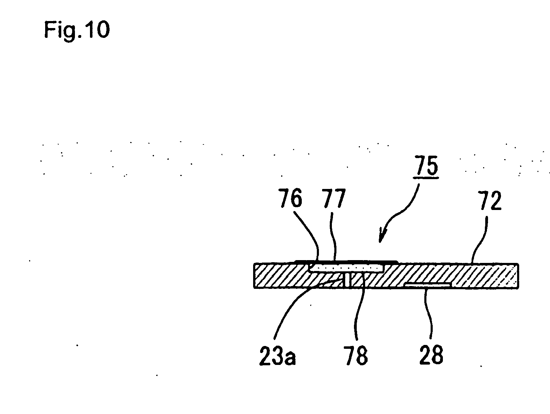

The biological component analyzer of still another embodiment is described below with reference to Figs. 9 and 10. The present embodiment mainly differs from the analyzer of the first embodiment in that a liquid container 75 is provided on a cartridge housing 72 rather than providing the syringe pump 25 on the analyzing unit 15. In the present embodiment, a description of the analyzing unit 15 is omitted, and only the biological component extraction cartridge is described. In Figs. 9 and 10 of the present embodiment, parts in common with Figs. 1 through 5 are designated by the same reference numbers.

As shown in Fig. 9, a liquid container 75 is provided at the end of the flow path 23 of a cartridge housing 72 in the present embodiment. The liquid container 75 includes a concavity 76 provided at the top surface of the cartridge housing 72, and a film 77 for covering the opening of the concavity 76, and a physiological saline 78 is accommodated by the concavity 76 and film 77. The liquid container 75 is connected to the flow path 23 through a connecting hole 23a, and plug 23b is provided within the flow path 23 to prevent the physiological saline 78 from flowing to the reaction reservoir 30 before the cartridge is used. The containment plug 23b, for example, may pack a high molecular weight substance, and is constructed so as to move the physiological saline 78 within the liquid container 75 to the reaction reservoir 30 by means of dissolving or deforming through heat, light, electricity, pressure or the like, when the cartridge is in use. The containment plug 23b is not inevitably necessary to prevent the physiological saline from entering the reaction reservoir 30 if the concavity 76 is sealed by the film 77.

When the cartridge is in use, the user peels away the film 77. Consequently, the physiological saline accommodated in the concavity 76 is pushed toward the reaction reservoir by atmosphere pressure.

Since the physiological saline 78 is accommodated beforehand in the biological component extraction cartridge in the biological component analyzer of the present embodiment, a pump, such as the syringe pump 25, is not required in the analyzing unit 15, thereby providing a biological component analyzer of simpler structure.

In the cartridge of the embodiment shown in Fig. 9, a cutter and a liquid capsule which is rupturable by the cutter and disposed below the cutter may be provided in the container 75, and physiological saline may be accommodated within in the liquid capsule. In this case, the user pushes the film 77 downward when using the cartridge. Consequently, the liquid capsule is ruptured by the cutter, and the physiological saline flows out from the liquid capsule. The discharged physiological saline then flows into the reaction reservoir 30 through the flow path 23.

Alternatively, a liquid supplying mechanism including the container 75, cutter, capsule, and flow path 23 may be provided in the analyzing unit 15 rather than the syringe pump 25 and flow path 23.

The container 75 and flow path 23 also may be omitted from the cartridge of the embodiment in Fig. 9, although such a modification is not in accordance with the claimed invention. In this case, a film may be removably adhered to the measuring surface 43 of the sensor member 19 exposed within the reaction reservoir 30, and physiological saline accommodated beforehand within the reaction reservoir 30, as one embodiment. According to this construction, the luminescence-producing reagent and enzymes (GOD and POD) applied to the measuring surface of the sensor member 19 separated from the physiological saline by the film. When the cartridge is to be used, the user brings the enzymes (GOD and POD) into contact with the physiological saline by removing the film from the sensor member 19. The film may also be removed by the user, or dissolved by thermal energy or light energy. A semi-solid substance such as a gel or the like may be used for a holding substance for holding glucose extracted from the subject as an alternative to the physiological saline.

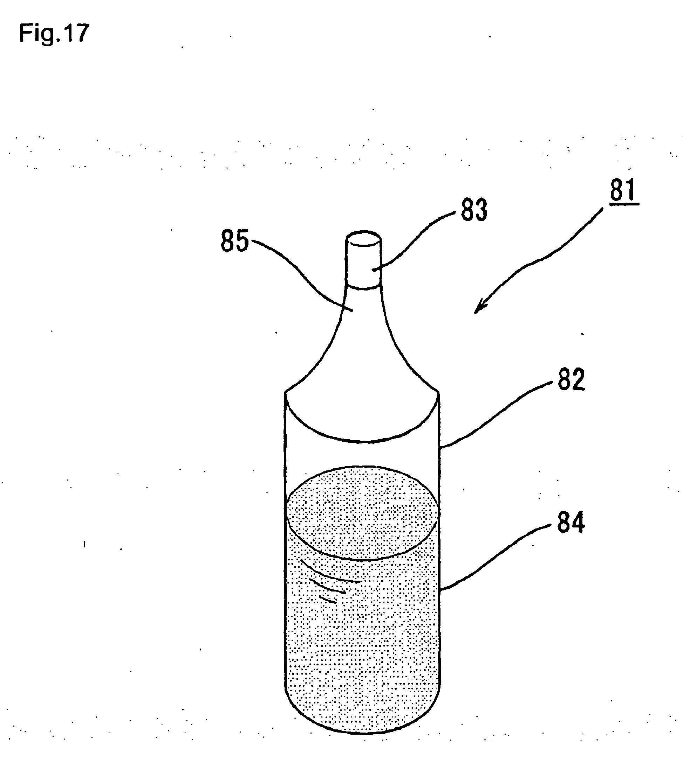

In the construction of the embodiment shown in Fig. 9 which omits the container 75 and flow path 23 from the cartridge, a container of physiological saline may be prepared, and the physiological saline may be injected into the reaction reservoir 30 through the second opening 20 using this container. An example of such a container is shown in Fig. 17. A container 81 includes a container body 82 and a cap 83, and physiological saline is accommodated in the container r81. The container body 82 is formed of a flexible resin. When in use, the user removes the cap 83, and inserts the mouth 85 of the container body 81 into the reaction reservoir 30 through the second opening 20, and squeezes the sides of the container body 82. Consequently, the physiological saline flows from the opening 85 and is supplied into the reaction reservoir 30. In this case, a member, such as a dried sponge, dried mesh sheet or the like is housed within the reaction reservoir 30 beforehand, such that the supplied physiological saline is retained by this member.

Furthermore, a dropper also may be used to supply the physiological saline from a container to the reaction reservoir 30.

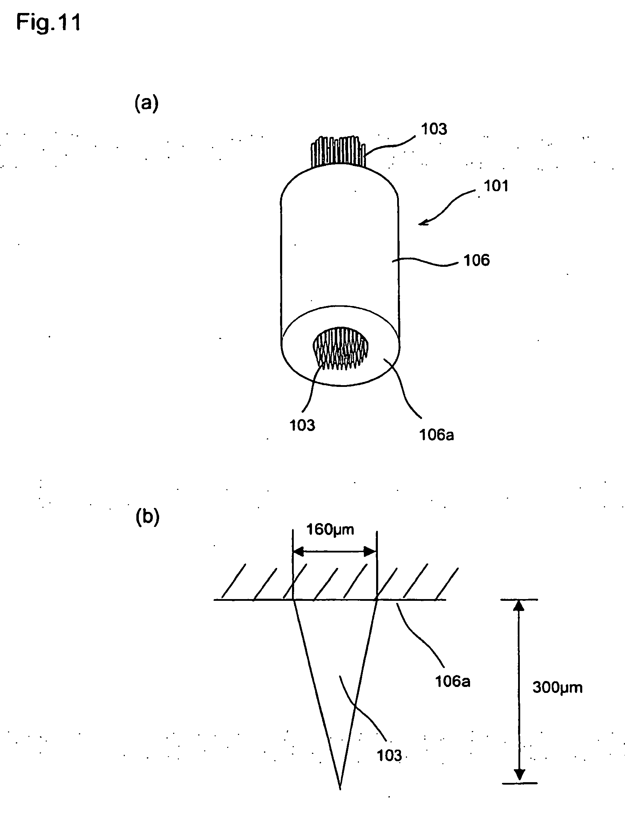

The glucose extraction and analysis method is described below based on Figs. 11 through 16.

In the extracting and analyzing method, the biological component analyzer 1 and the micro needle 101 shown in Fig. 1 and 11 are used.

As shown in Fig. 11(a), the micro needle 101 is constructed as a plurality of needles 103 bundled in a holder 106; thirty needles 103 are used, and the holder 106 has an external diameter of 10 mm, internal diameter of 3 mm, and the length of the needles 103 extending from the endface 106a of the holder 106 is 300 pm.

The part of the needle 103 extending from the endface 106a is conical, as shown in Fig. 11(b), and the part nearest the endface 106a, that is, the diameter of the base of the cone, is 160 µm.

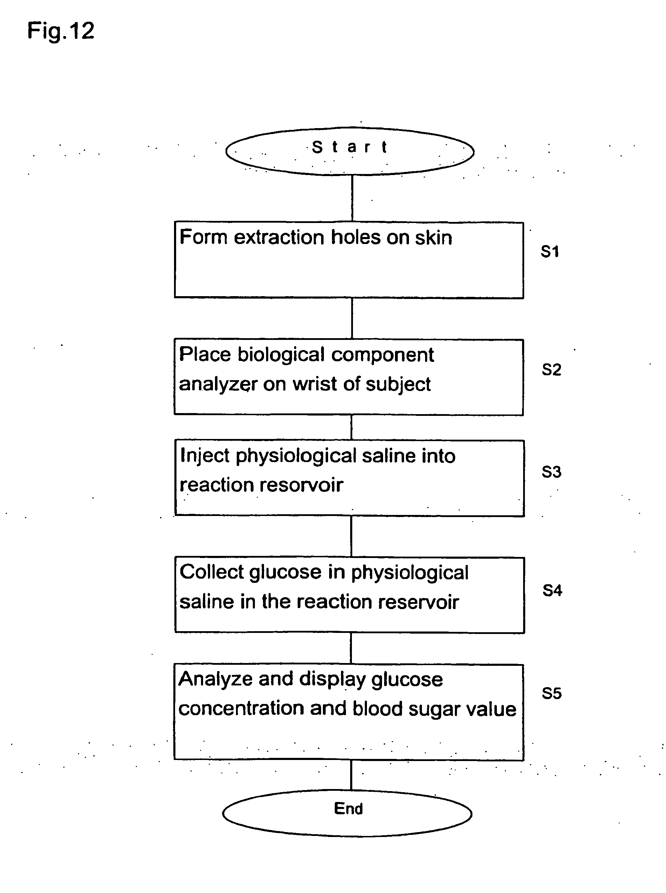

The analyzing method is described below referring to Fig. 12.

In step S1, the user forms extraction holes by pressing the plurality of needles 103 of an insertion device 101 against the skin of the wrist.

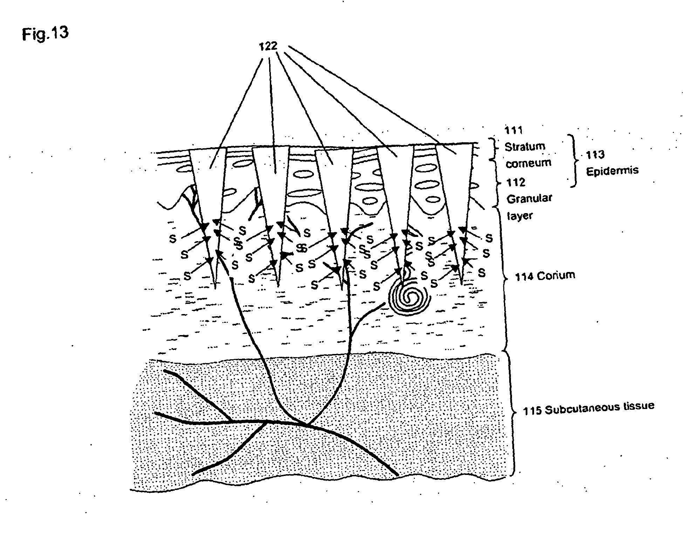

Fig. 13 schematically shows cross section of the skin in which the extraction holes are formed. As indicated in the drawing, epidermis 113 is the outermost surface of the skin in contact with the air. The epidermis 113 includes the stratum corneum 111 which has a high electrical resistance, and granular layer 112 disposed below the stratum corneum 111. The corium 114 is below the epidermis 113, and subcutaneous tissue 115 is below the corium 114. A plurality of extraction holes 122, which are formed by pressing the plurality of needles 103 of the insertion device 101 against the skin, pass through the epidermis 113, that is, the stratum corneum 111 and granular layer 112, and reach midway of the corium 114, but do not reach the subcutaneous tissue 115. The extraction holes 122 have the widest diameter at the skin surface, and the narrowest diameter near the subcutaneous tissue 115. The diameter of the extraction hole 122 at the skin surface is approximately 160 µm, and the depth of the extraction hole 122 is approximately 300µm. When the extraction holes 122 are formed in step S1, body fluid saturating the corium 114 is extracted in the extraction holes 122, as indicated by the arrow S. This body fluid contains glucose.

In step S2, the user mounts the biological component analyzer 1 on the wrist, as shown in Fig. 1(b). At this time, the second opening 20 (refer to Fig. 4) is aligned with the location on the skin at which the extraction holes 122 were formed in step S1.

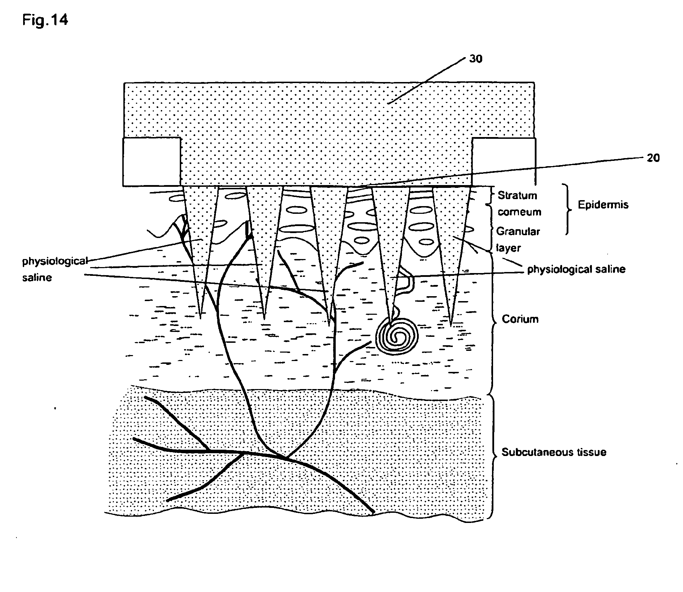

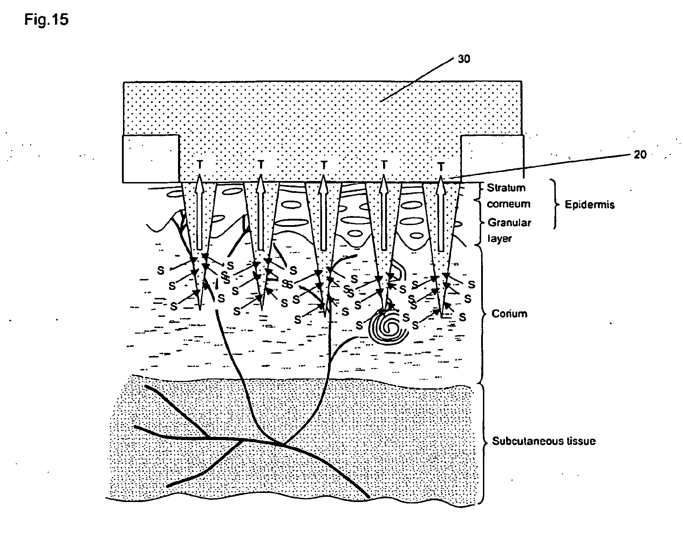

In step S3, the user pushes the piston 26 and moves the physiological saline from the syringe pump 25 into the reaction reservoir 30. Consequently, the physiological saline fills the reaction reservoir 30, and some of the physiological saline flows from the reaction reservoir 30 through the second opening 20 into the extraction holes 122, as shown in Fig. 14. When the physiological saline flows into the extraction holes 122, the body fluid extracted in the extraction holes 122 due to the formation of the extraction holes 122 in step S1 moves in the direction of the physiological saline in the reaction reservoir 30(T direction in Fig. 15), as shown in Fig. 15. Since the concentration of body fluid is low relative to the physiological saline in the extraction holes 122, the body fluid is extracted into the physiological saline in the extraction holes 122, as indicated by the arrow S.

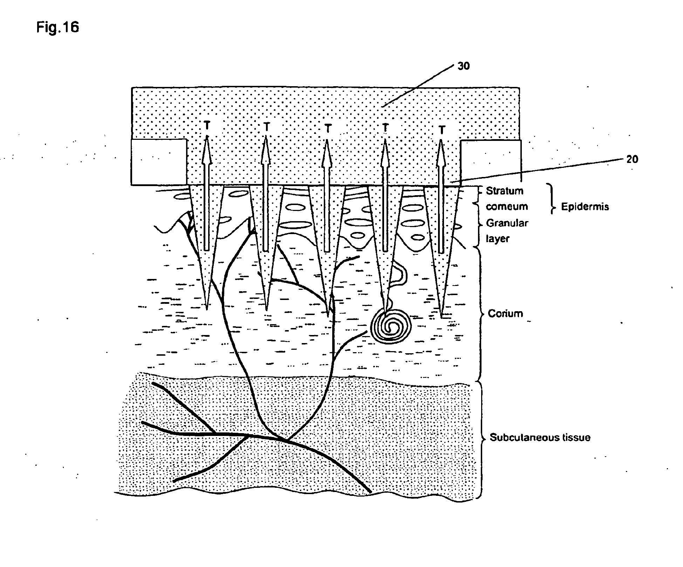

In step S4, a predetermined voltage (0.8 V) from the constant-voltage power supply 32 is applied to the extraction electrode 13 for a predetermined time (3 minutes). Consequently, since the body fluid extracted in the extraction holes 122 is charged, the body fluid is facilitated in moving in the direction of the reaction reservoir 30 (T direction in Fig. 16) under the influence of the electrical charge. Although the glucose contained in the body fluid is not charged, this glucose moves in the T direction in conjunction with the movement of the other components which are charged.

In step S5, an electrical signal which reflects the glucose concentration is output by the photoreceptor element 36 as described previously, and the controller 31 calculates the glucose concentration based on the received signal, and a blood sugar value is calculated from the calculated glucose concentration. The calculated glucose concentration and blood sugar value are displayed on the display unit 38.

Although the previously described embodiments have been described in terms of a construction wherein a gel containing enzymes and luminescence-producing reagent is applied on and dried on the measuring surface 43 of the sensor member 19, the present invention is not limited to this construction inasmuch as alternative constructions include those in which enzymes and luminescence-producing reagent solidified by cross-linking macromolecules such as optical cross-linking polyvinyl alcohols and the like, and those in which enzymes are solidified by well known fatty molecules having molecular structures manifesting luminescence-producing functionality. Furthermore, the enzyme may be applied beforehand on the measuring surface 43 of the sensor member 19, and the luminescence-producing reagent may be dissolved in the physiological saline.

In the above embodiments, the enzymes and luminescence-producing reagents are applied in a dried state on the measuring surface 43 of the sensor member 19, however, the present invention is not limited to this mode inasmuch as the enzymes and luminescence-producing reagent may be applied in a dried state on an inner wall of the reaction reservoir 30.

Although the above embodiments have been described by way of examples of applying a biological component extraction cartridge to a biological component analyzer for detecting glucose concentration based on an optical signal, the present invention is not limited to this mode inasmuch as the present invention may be applied to biological component analyzers for detecting glucose based on electrical signals using an enzyme electrode method.

In the above embodiments, the electrically conductive fluid such as physiological saline is used to retain the glucose extracted from a subject, the present invention is not limited to this mode inasmuch as a nonconductive fluid such as purified water also may be used. In this case, both the extraction electrode and positive electrode may be disposed in the reaction reservoir 30 or extraction reservoir 50.

Although the above embodiments have been described in terms of a biological component analyzer which calculates glucose concentration and blood sugar value as the analysis result, the present invention is not limited to this mode inasmuch as the present invention also may be applied to biological component analyzers which calculate biochemical item, such as lactic acid, uric acid, cholesterol and the like, as the analysis result.

## Claims

1. An analyzer (2) for extracting and analyzing an analyte from a subject, the analyzer comprising:
a reaction reservoir (30) capable of holding a liquid for holding the analyte extracted from the subject;
a liquid supplying mechanism (25, 75);
an extraction reservoir (50) connected to the liquid supplying mechanism, and capable of holding the liquid for holding the analyte extracted from the subject;
the liquid supplying mechanism (25, 75) for holding a liquid to be supplied to the extraction reservoir (50) and for supplying the liquid to the extraction reservoir;
a first flow path (23, 24) connecting the liquid supplying mechanism (25) and the extraction reservoir (50); **characterized by**:
a second flow path (18a) connecting the extraction reservoir (50) and the reaction reservoir (30);
the liquid supplying mechanism for supplying the liquid to the reaction reservoir (30) through the extraction reservoir (50), arranged so that:
the analyte is extracted from the subject to the extraction reservoir (50) when the liquid supplying mechanism (25, 75) has supplied liquid to the extraction reservoir (50) and is moved to the reaction reservoir (30) together with the liquid supplied to the extraction reservoir through the second flow path (18a), and
wherein the reaction reservoir holds an enzyme which is a catalyst for the analyte in a dry state.

2. The analyzer of Claim 1, further comprising:
a detection unit (19) for detecting an analyte held in the liquid supplied to the reaction reservoir (30) by the liquid supplying mechanism (25, 75).

3. The analyzer of Claim 2, wherein the detection unit (19) includes a sensor member (43) for supplying analyte through the liquid and disposed so as to be exposed within the reaction reservoir; and
a signal output device (36) for obtaining a signal based on the analyte through the sensor member and outputting the signal.

4. The analyzer of Claim 3, wherein the signal output device includes a light source (34) for irradiating the sensor member with light; and a photodetector (36) for detecting the light irradiated from the light source through the sensor member, and outputting signals based on the detected light.

5. The analyzer of claim 3 or 4, wherein the part of the sensor member exposed within the reaction reservoir is a measuring surface (43) having applied thereon the enzyme in the dry state.

6. The analyzer of any one of the preceding claims,
wherein the analyte is glucose.

7. The analyzer of any one of the preceding claims,
wherein the liquid supplying mechanism includes a rupturable liquid container (75) for accommodating the liquid; a rupturing tool (77) for rupturing the liquid container; and said first flow path (23) for directing liquid flowing from the
liquid container to the extraction reservoir (50) when the liquid container is ruptured by the rupturing tool.

8. A cartridge (51) detachably mountable to an analyzer (2) for extracting and analyzing an analyte from a subject, the cartridge comprising:
a reaction reservoir (30) capable of holding a liquid for holding the analyte extracted from the subject;
a liquid supplying mechanism (25, 75);
an extraction reservoir (50) connected to the liquid supplying mechanism, and capable of holding the liquid for holding the analyte extracted from the subject;
the liquid supplying mechanism (25, 75) for holding a liquid to be supplied to the extraction reservoir (50), and for supplying the liquid to the extraction reservoirs (50);
wherein the cartridge comprises a first flow path (23, 24) between the extraction reservoir and the liquid supplying mechanism when the cartridge is mounted to the analyzer; **characterized by**:
a second flow path (18a) connecting the extraction reservoir and the reaction reservoir;
the liquid supplying mechanism for supplying the liquid to the reaction reservoir through the extraction reservoir; arranged so that:
the analyte is extracted from the subject to the extraction reservoir when supplied with liquid and is moved to the reaction reservoir together with the liquid supplied to the extraction reservoir through the second flow path; and
wherein the reaction reservoir holds an enzyme which is a catalyst for the analyte in a dry state.

9. The cartridge of Claim 8, wherein the liquid supplying mechanism includes a rupturable liquid container (75) for accommodating the liquid; a rupturing tool (77) for rupturing the liquid container; and said first flow path (23) for directing liquid flowing from the liquid container to the reservoir when the liquid container is ruptured by the rupturing tool.

10. The analyzer of any one of claims 1 to 7, comprising an extraction electrode (13) and a constant voltage power supply (32) for applying an electric field to the subject so as to extract analyte from the subject to the holding liquid in the extraction reservoir.

11. The cartridge of claim 8 or 9, comprising an extraction electrode (13) for applying an electric field to the subject so as to extract analyte from the subject to the holding liquid in the extraction reservoir.

12. The analyzer of any one of claims 1 to 7, wherein
the liquid supplying mechanism includes a pump (25) for discharging the liquid.

## Patentansprüche

1. Analysevorrichtung (2) zum Extrahieren und Analysieren eines Analyts von einem Untersuchungsobjekt, die Analysevorrichtung aufweisend:
ein Reaktionsreservoir (30), das eine Flüssigkeit zum Aufnehmen des von dem Untersuchungsobjekt extrahierten Analyts aufnehmen kann;
ein Flüssigkeitszuführmechanismus (25, 75);
ein Extrahierungsreservoir (50), das mit dem Flüssigkeitszuführmechanismus verbunden ist und die Flüssigkeit zum Aufnehmen des von dem Untersuchungsobjekt extrahierten Analyts aufnehmen kann;
der Flüssigkeitszuführmechanismus (25, 75) zur Aufnahme einer dem Extrahierungsreservoir (50) zuzuführenden Flüssigkeit und zum Zuführen der Flüssigkeit zu dem Extrahierungsreservoir;
einen ersten Durchflussweg (23, 24), der den Flüssigkeitszuführmechanismus (29) und das Extrahierungsreservoir (50) verbindet; **gekennzeichnet durch**:
einen zweiten Durchflussweg (18a), der das Extrahierungsreservoir (50) und das Reaktionsreservoir (30) verbindet;
wobei der Flüssigkeitszuführmechanismus zum Zuführen der Flüssigkeit **durch** das Extrahierungsreservoir (50) zu dem Reaktionsreservoir (30) so angeordnet ist, dass:
der Analyt von dem Untersuchungsobjekt zu dem Extrahierungsreservoir (50) extrahiert wird, wenn der Flüssigkeitszuführmechanismus (25, 75) Flüssigkeit zu dem Extrahierungsreservoir (50) zugeführt hat und zu dem Reaktionsreservoir (30) bewegt wird, zusammen mit der **durch** den zweiten Durchflussweg (18a) zu dem Extrahierungsreservoir zugeführten Flüssigkeit; und
wobei das Reaktionsreservoir ein Enzym in einem trockenen Zustand aufweist, das ein Katalysator für den Analyt ist.

2. Analysevorrichtung gemäß Anspruch 1, weiterhin aufweisend:
eine Nachweiseinheit (19) zum Nachweisen eines in der Flüssigkeit aufgenommenen Analyts, das durch den Flüssigkeitszuführmechanismus (25, 75) dem Reaktionsreservoir (30) zugeführt wird.

3. Analysevorrichtung gemäß Anspruch 2, bei der die Nachweiseinheit (19) ein Sensorteil (43) für das Zuführen von Analyt durch die Flüssigkeit aufweist, das so angeordnet ist, um innerhalb des Reaktionsreservoirs ausgesetzt zu sein; und
eine Signalausgabeeinrichtung (36) zum Erhalten eines auf dem Analyt basierenden Signals über das Sensorteil und Ausgeben des Signals.

4. Analysevorrichtung gemäß Anspruch 3, bei der die Signalausgabeeinrichtung eine Lichtquelle (34) zum Anstrahlen des Sensorteils mit Licht aufweist; und einen Photodetektor (36) zum Detektieren des von der Lichtquelle über das Sensorteil ausgestrahlten Lichts, und Ausgeben des auf dem detektierten Licht basierenden Signals.

5. Analysevorrichtung gemäß Anspruch 3 oder 4, bei der der Teil des in dem Reaktionsreservoir ausgesetzten Sensorteils eine Messfläche (43) ist, auf der das Enzym in dem trockenem Zustand aufgebracht ist.

6. Analysevorrichtung gemäß einem der vorstehenden Ansprüche, bei der der Analyt Glukose ist.

7. Analysevorrichtung gemäß einem der vorstehenden Ansprüche, bei der der Flüssigkeitszuführmechanismus einen aufreißbaren Flüssigkeitsbehälter (75) zum Aufnehmen der Flüssigkeit aufweist; ein Aufreißwerkzeug (77) zum Aufreißen des Flüssigkeitsbehälters; und den ersten Durchflussweg (23), um Flüssigkeit zu leiten, die von dem Flüssigkeitsbehälter zu dem Extrahierungsreservoir (50) fließt, wenn der Flüssigkeitsbehälter durch das Aufreißwerkzeug aufgerissen wird.

8. Patrone (51), die abnehmbar an einer Analysevorrichtung (2) zum Extrahieren und Analysieren eines Analyts von einem Untersuchungsobjekt eingesetzt ist, die Patrone aufweisend:
ein Reaktionsreservoir (30), das eine Flüssigkeit zum Aufnehmen des von dem Untersuchungsobjekt extrahierten Analyts aufnehmen kann;
ein Flüssigkeitszuführmechanismus (25, 75);
ein Extrahierungsreservoir (50), das mit dem Flüssigkeitszuführmechanismus verbunden ist und die Flüssigkeit zum Aufnehmen des von dem Untersuchungsobjekt extrahierten Analyts aufnehmen kann;
der Flüssigkeitszuführmechanismus (25, 75) zur Aufnahme einer dem Extrahierungsreservoir (50) zuzuführenden Flüssigkeit und zum Zuführen der Flüssigkeit zu dem Extrahierungsreservoir (50);
wobei die Patrone einen ersten Durchflussweg (23, 24) zwischen dem Extrahierungsreservoir und dem Flüssigkeitszuführmechanismus aufweist, wenn die Patrone in die Analysevorrichtung eingesetzt ist; **gekennzeichnet durch**:
einen zweiten Durchflussweg (18a), der das Extrahierungsreservoir und das Reaktionsreservoir verbindet;
wobei der Flüssigkeitszuführmechanismus zum Zuführen der Flüssigkeit **durch** das Extrahierungsreservoir zu dem Reaktionsreservoir so angeordnet ist, dass:
der Analyt von dem Untersuchungsobjekt zu dem Extrahierungsreservoir wenn Flüssigkeit zugeführt wird extrahiert wird und zu dem Reaktionsreservoir bewegt wird, zusammen mit der durch den zweiten Durchflussweg zu dem Extrahierungsreservoir zugeführten Flüssigkeit; und
wobei das Reaktionsreservoir ein Enzym in einem trockenen Zustand aufweist, das ein Katalysator für den Analyt ist.

9. Patrone gemäß Anspruch 8, bei der der Flüssigkeitszuführmechanismus einen aufreißbaren Flüssigkeitsbehälter (75) zur Aufnahme der Flüssigkeit aufweist; ein Aufreißwerkzeug (77) zum Aufreißen des Flüssigkeitsbehälters; und den ersten Durchflussweg (23) zum Leiten der Flüssigkeit, die von dem Flüssigkeitsbehälter zu dem Reservoir fließt, wenn der Flüssigkeitsbehälter durch das Aufreißwerkzeug aufgerissen wird.

10. Analysevorrichtung gemäß einem der Ansprüche 1 bis 7, aufweisend eine Extrahierungselektrode (13) und eine konstante Spannungsquelle (32) zum Anlegen eines elektrischen Feldes an dem Untersuchungsobjekt, um so Analyt von dem Untersuchungsobjekt zu der aufnehmenden Flüssigkeit in dem Extrahierungsreservoir zu extrahieren.

11. Patrone gemäß Anspruch 8 oder 9, aufweisend eine Extrahierungselektrode (13) zum Anlegen eines elektrischen Feldes an dem Untersuchungsobjekt, um so Analyt von dem Untersuchungsobjekt zu der aufnehmenden Flüssigkeit in dem Extrahierungsreservoir zu extrahieren.

12. Analysevorrichtung gemäß einem der Ansprüche 1 bis 7, bei der der Flüssigkeitszuführmechanismus eine Pumpe (25) zum Ablassen der Flüssigkeit aufweist.

## Revendications

1. Analyseur (2) destiné à extraire et à analyser une analyte d'un sujet, l'analyseur comprenant :
un réservoir de réaction (30) pouvant maintenir un liquide pour maintenir l'analyte extraite du sujet ;
un mécanisme (25, 75) d'alimentation en liquide ;
un réservoir d'extraction (50) relié au mécanisme d'alimentation en liquide, et pouvant maintenir le liquide pour maintenir l'analye extraite du sujet ;
le mécanisme (25, 75) d'alimentation en liquide étant destiné à maintenir un liquide à alimenter au réservoir d'extraction (50) et à alimenter le liquide au réservoir d'extraction ;
un premier chemin d'écoulement (23, 24) reliant le mécanisme (25) d'alimentation en liquide et le réservoir d'extraction (50) ; **caractérisé par** :
un deuxième chemin d'écoulement (18a) reliant le réservoir d'extraction (50) et le réservoir de réaction (30) ;
le mécanisme d'alimentation en liquide étant destiné à alimenter le liquide au réservoir de réaction (30) à travers le réservoir d'extraction (50), agencé de sorte que :
l'analyte est extraite du sujet au réservoir d'extraction (50) lorsque le mécanisme (25, 75) d'alimentation en liquide a alimenté du liquide au réservoir d'extraction (50) et est déplacée vers le réservoir de réaction (30) ensemble avec le liquide alimenté au réservoir d'extraction à travers le deuxième chemin d'écoulement (18a) ; et
où le réservoir de réaction maintient une enzyme qui est un catalyseur pour l'analyte à un état sec.

2. Analyseur de la revendication 1, comprenant en outre :
une unité de détection (19) destinée à détecter une analyte maintenue dans le liquide alimenté au réservoir de réaction (30) par le mécanisme (25, 75) d'alimentation en liquide.

3. Analyseur de la revendication 2, dans lequel l'unité de détection (19) comporte un élément capteur (43) destiné à alimenter une analyte à travers le liquide et disposé de sorte à être exposé dans le réservoir de réaction ; et
un dispositif (36) de sortie de signal destiné à obtenir un signal sur la base de l'analyte à travers l'élément capteur et à délivrer en sortie le signal.

4. Analyseur de la revendication 3, dans lequel le dispositif de sortie de signal comporte une source lumineuse (34) destinée à irradier l'élément capteur avec de la lumière ; et un photo-détecteur (36) destiné à détecter la lumière irradiée de la source lumineuse à travers l'élément capteur, et à délivrer en sortie des signaux sur la base de la lumière détectée.

5. Analyseur de la revendication 3 ou 4, dans lequel la partie de l'élément capteur exposé dans le réservoir de réaction est une surface de mesure (43) ayant appliqué sur celui-ci l'enzyme à l'état sec.

6. Analyseur de l'une quelconque des revendications précédentes, dans lequel l'analye est du glucose.

7. Analyseur de l'une quelconque des revendications précédentes, dans lequel le mécanisme d'alimentation en liquide comporte un conteneur (75) de liquide pouvant se rompre, destiné à accueillir le liquide ; un outil de rupture (77) destiné à rompre le conteneur de liquide ; et ledit premier chemin d'écoulement (23) destiné à diriger le liquide s'écoulant du conteneur de liquide au réservoir d'extraction (50) lorsque le conteneur de liquide est rompu par l'outil de rupture.

8. Cartouche (51) pouvant être montée de manière détachable sur un analyseur (2) destiné à extraire et à analyser une analyte d'un sujet, la cartouche comprenant :
un réservoir de réaction (30) pouvant maintenir un liquide pour maintenir l'analyte extraite du sujet ;
un mécanisme (25, 75) d'alimentation en liquide ;
un réservoir d'extraction (50) relié au mécanisme d'alimentation en liquide, et pouvant maintenir le liquide pour maintenir l'analye extraite du sujet ;
le mécanisme (25, 75) d'alimentation en liquide étant destiné à maintenir un liquide à alimenter au réservoir d'extraction (50), et à alimenter le liquide au réservoir d'extraction (50) ;
où la cartouche comprend un premier chemin d'écoulement (23, 24) entre le réservoir d'extraction et le mécanisme d'alimentation en liquide lorsque la cartouche est montée sur l'analyseur ;
**caractérisée par**
un deuxième chemin d'écoulement (18a) reliant le réservoir d'extraction et le réservoir de réaction ;
le mécanisme d'alimentation en liquide étant destiné à alimenter le liquide au réservoir de réaction à travers le réservoir d'extraction, agencé de sorte que :
l'analyte est extraite du sujet au réservoir d'extraction lorsque celui-ci est alimenté avec le liquide et est déplacée vers le réservoir de réaction ensemble avec le liquide alimenté au réservoir d'extraction à travers le deuxième chemin d'écoulement ; et
où le réservoir de réaction maintient une enzyme qui est un catalyseur pour l'analyte à un état sec.

9. Cartouche de la revendication 8, dans laquelle le mécanisme d'alimentation en liquide comporte un conteneur (75) de liquide pouvant se rompre destiné à accueillir le liquide ; un outil de rupture (77) destiné à rompre le conteneur de liquide ; et ledit premier chemin d'écoulement (23) destiné à diriger le liquide s'écoulant du conteneur de liquide au réservoir de réaction (50) lorsque le conteneur de liquide est rompu par l'outil de rupture.

10. Analyseur de l'une quelconque des revendications 1 à 7, comprenant une électrode d'extraction (13) et une alimentation électrique (32) à tension constante pour appliquer un champ électrique au sujet de sorte à extraire une analyte du sujet au liquide de maintien dans le réservoir d'extraction.

11. Cartouche de la revendication 8 ou 9, comprenant une électrode d'extraction (13) destinée à appliquer un champ électrique au sujet de sorte à extraire une analyte du sujet au liquide de maintien dans le réservoir d'extraction.

12. Analyseur de l'une quelconque des revendications 1 à 7, dans lequel le mécanisme d'alimentation en liquide comporte une pompe (25) destinée à décharger le liquide.
